(19) Europäisches Patentamt
European Patent Office
Office européen des brevets

(11) Veröffentlichungsnummer: **0 015 227**
**B1**

# (12) EUROPÄISCHE PATENTSCHRIFT

(45) Veröffentlichungstag der Patentschrift: **18.05.83**

(51) Int. Cl.³: **C 07 D 209/52,** A 61 K 31/40 // A61K31/557, C07C177/00, C07D309/12

(21) Anmeldenummer: **80730010.8**

(22) Anmeldetag: **15.02.80**

## (54) Neue Prostacyclin-Derivate und Verfahren zu ihrer Herstellung.

(30) Priorität: **20.02.79 DE 2907118**

(43) Veröffentlichungstag der Anmeldung: **03.09.80 Patentblatt 80/18**

(45) Bekanntmachung des Hinweises auf die Patenterteilung: **18.05.83 Patentblatt 83/20**

(84) Benannte Vertragsstaaten: **AT BE CH DE FR GB IT LU NL SE**

(56) Entgegenhaltungen:
**DE-A-2 118 686**
**DE-A-2 517 801**
**FR-A-2 204 422**

**TETRAHEDRON LETTERS, Nr. 16, April 1978, Pergamon Press, GB, G.L. BUNDY et al.: "The synthesis of *nitrogen-containing* prostacyclin analogs", Seiten 1371–1374**
**M. NEGWER: Organ.-chem. Arzneimittel, Berlin 1978, S. 1251**
**Fortschritte in d. Arzneimittelforschung, Basel 1973, Bd. 17, S. 469**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Vorbrüggen, Helmut, Prof., Wilkestrasse 7, D-1000 Berlin 27 (DE)**
Erfinder: **Radüchel, Bernd, Dr., Gollanczstrasse 132, D-1000 Berlin 28 (DE)**
Erfinder: **Skuballa, Werner, Dr., Olwenstrasse 25, D-1000 Berlin 28 (DE)**
Erfinder: **Mannesmann, Gerda, Dr., Hagenplatz 3e, D-1000 Berlin 33 (DE)**
Erfinder: **Losert, Wolfgang, Prof., Niedstrasse 12, D-1000 Berlin 41 (DE)**
Erfinder: **Casals, Jorge, Dr., Wicherstrasse 20, D-1000 Berlin 33 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 015 227 B1

ACTORUM AG

# Neue Prostacyclin-Derivate und Verfahren zu ihrer Herstellung

Beschreibung:

Die Erfindung betrifft neue Prostacyclin-Derivate, Verfahren zu ihrer Herstellung sowie ihre Verwendung als Arzneimittel.

Prostacyclin ($PGI_2$), einer der Hauptfaktoren bei der Blutplättchenaggregation, wirkt dilatierend auf verschiedene Blutgefässe (Science 196, 1072) und ist daher als Mittel zur Blutdrucksenkung anzusehen. $PGI_2$ besitzt jedoch nicht die für ein Arzneimittel notwendige Stabilität. So beträgt die Halbwertzeit von $PGI_2$ bei physiologischen pH-Werten und bei Raumtemperatur nur wenige Minuten.

In einer Publikation der Firma Upjohn (G.L. Bundy et al.), Tetrahedron Letters 1978, 1371) sowie in der DE-OS 2 826 096 wird über 9α,6-Nitrilo-prostaglandine berichtet.

Die Azaprostacycline aus Tetrahedron Letters, 1978, 1371 enthalten weder eine 18-In-Gruppierung noch eine 16,17-Substitution und wirken verstärkend gegen die Noradrenalin-induzierte Kontraktion am Kaninchen, während die erfindungsgemässen Azaprostacycline in diesem Test abschwächend wirken.

Aus M. Negwer (Organ.-chem. Arzneimittel und ihre Synonyma), 5. Auflage, Akademie-Verlag Berlin, 1978, S. 1251, und aus Fortschritte der Arzneimittelforschung (E. Jucker), Vol. 17, Bd. 17, S. 469 (1973) sind Prostaglandine mit einer 13-In-Struktur bzw. 16,16-Dimethyl-Substitution bekannt.

In DE-OS 2 118 686, DE-OS 2 517 801 und FR 2 204 422 werden Prostaglandine beschrieben, die entweder in 18- oder 16-Stellung eine In-Gruppierung aufweisen oder in 15-Stellung mit einer Acetylengruppe substituiert sind.

Es wurde nun gefunden, dass durch Einführung von Dreifachbindungen und gegebenenfalls Alkylgruppen in der unteren Kette des 9-Desoxy-9α,6-nitrilo-PGF eine längere Wirkungsdauer, grössere Selektivität und bessere Wirksamkeit erzielt werden kann.

Die erfindungsgemässen Verbindungen wirken blutdrucksenkend und bronchodilatorisch. Sie sind ausserdem zur Inhibierung der Thrombozytenaggregation geeignet.

Die Erfindung betrifft Prostanderivate der allgemeinen Formel I

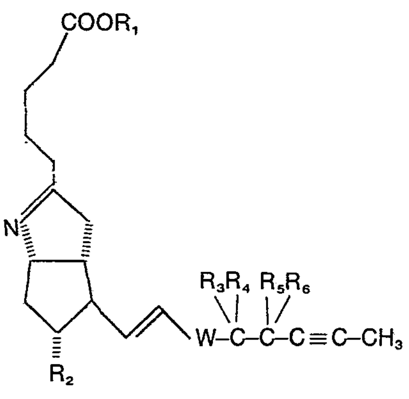

I

worin

$R_1$ Wasserstoff, Alkyl mit 1–4 C-Atomen, Cycloalkyl mit 5 oder 6 C-Atomen oder Phenyl,

W eine freie oder durch Tetrahydropyranyl substituierte Hydroxymethylengruppe oder eine freie oder durch Tetrahydropyranylsubstituierte 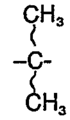 Gruppe, wobei die OH-Gruppe α-ständig ist und $R_3$, $R_4$, $R_5$, $R_6$ ein Wasserstoffatom oder eine Alkylgruppe mit 1–5 C-Atomen,

$R_2$ eine freie oder durch Tetrahydropyranyl substituierte Hydroxygruppe und falls $R_1$ die Bedeutung eines Wasserstoffatoms hat, deren Salze mit physiologisch verträglichen Basen bedeuten.

Als Alkylgruppe $R_1$ sind gerade oder verzweigte Alkylgruppen mit 1–4 C-Atomen zu betrachten, wie beispielsweise Methyl, Äthyl, Propyl, Butyl, Isobutyl, tert.-Butyl.

Die Cycloalkylgruppe $R_1$ kann im Ring 5 und 6 Kohlenstoffatome enthalten. Die Ringe können durch Alkylgruppen mit 1–4 Kohlenstoffatomen substituiert sein. Beispielsweise seien genannt Cyclopentyl-, Cyclohexyl, Methylcyclohexyl.

Die Hydroxygruppen $R_2$ und in W können funktionell abgewandelt sein, beispielsweise durch Verätherung oder Veresterung, wobei die freien oder abgewandelten Hydroxygruppen in W α- oder β-ständig sein können, wobei freie Hydroxygruppen bevorzugt sind. Als Äther- und Acylreste kommen die dem Fachmann bekannten Reste in Betracht. Bevorzugt sind leicht abspaltbare Ätherreste wie beispielsweise der Tetrahydropyranyl-, Tetrahydrofuranyl-, α-Äthoxyäthyl-, Trimethylsilyl-, Dimethyl-tert.-butylsilyl- und Tribenzyl-silylrest. Als Acylreste kommen beispielsweise in Frage: Acetyl, Propionyl, Butyryl, Benzoyl.

Als Alkylgruppen $R_3$, $R_4$, $R_5$, $R_6$ kommen gerad- und verzweigtkettige Alkylreste mit 1–5 Kohlenstoffatomen in Betracht wie beispielsweise der Methyl-, Äthyl-, Propyl-, Isopropyl-, Butyl-, Isobutyl-, tert.-Butyl-, Pentyl, Isopentyl- und Neopentyl-Rest. Bevorzugt sind die Methyl- und die Äthylgruppe.

Zur Salzbildung mit den freien Säuren ($R_1 = H$) sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemässen Prostanderivate der allgemeinen Formel I, dadurch gekennzeichnet, dass man in an sich bekannter Weise eine Verbindung der allgemeinen Formel II,

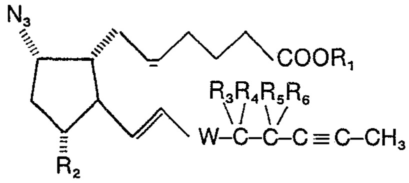

II

worin $R_1$, $R_2$, $R_3$, $R_4$, $R_5$, $R_6$ und W die obengenannten Bedeutungen haben in einem inerten Lösungsmittel einer thermischen Behandlung unterwirft und gegebenenfalls anschliessend in beliebiger Reihenfolge geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe mit einer physiologisch verträglichen Base in ein Salz überführt.

Die thermische Umsetzung der Verbindung der allgemeinen Formel II wird bei Temperaturen von 20–150 °C, vorzugsweise 40–120 °C, vorgenommen. Für den bevorzugten Temperaturbereich kommen beispielsweise folgende inerte Lösungsmittel in Frage: Essigsäureäthylester, Essigsäuremethylester, Tetrahydrofuran, Dimethoxyäthan, Tetrachlorkohlenstoff, Methylenchlorid, 1,2-Dichloräthan, Dimethylformamid usw.

Die Verseifung der Prostaglandinester wird nach den dem Fachmann bekannten Methoden durchgeführt, beispielsweise mit basischen Katalysatoren. Die Einführung der Estergruppe, bei welcher $R_1$ eine Alkylgruppe mit 1–10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, dass man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie z.B. Methylenchlorid, vermischt. Nach beendeter Umsetzung in 1 bis 30 Minuten wird das Lösungsmittel entfernt und der Ester in üblicher Weise gereinigt. Diazolkane sind entweder bekannt oder können nach bekannten Methoden hergestellt werden [Org. Reactions Bd. 8, Seiten 389–394 (1954)].

Die Einführung der Estergruppe $R_1$, bei welcher $R_1$ eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin oder Triäthylamin, in einem inerten Lösungsmittel umgesetzt. Als Lösungsmittel kommen Methylenchlorid, Äthylenchlorid, Chloroform, Essigester, Tetrahydrofuran, vorzugsweise Chloroform, in Frage. Die Reaktion wird bei Temperaturen zwischen −30 und +50 °C, vorzugsweise bei +10 °C, durchgeführt.

Die Prostaglandin-Derivate der allgemeinen Formel I mit $R_1$ in der Bedeutung eines Wasserstoffatoms können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Zur Herstellung eines Aminsalzes, die in üblicher Weise erfolgt, wird die PG-Säure zum Beispiel in einem geeigneten Lösungsmittel, beispielsweise Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuss angewandt, vorzugsweise in der 2- bis 10fachen Menge des theoretischen Bedarfs. Die Umsetzung ist normalerweise bei 0–30 °C nach 15–30 Minuten beendet. Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wässrigen Lösung einer organischen Säure, wie zum Beispiel Essigsäure, Propionsäure u.a., durchgeführt. Zur Verbesserung der Löslichkeit wird zweckmässigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20 und 80 °C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0 und 80 °C durchgeführt.

Die Verseifung der Acylgruppen erfolgt beispielsweise mit Alkali- oder Erdalkalicarbonaten oder -hydroxyden in einem Alkohol oder der wässrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium-und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei −10 bis 70 °C, vorzugsweise bei 25 °C.

Das als Ausgangsmaterial für das vorstehend beschriebene Verfahren verwendete Azid der allgemeinen Formel II kann hergestellt werden, indem man einen Alkohol der allgemeinen Formel III

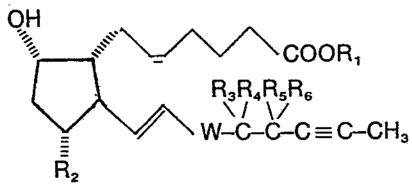
III

(DE-OS 2 729 960), worin freie Hydroxygruppen in $R_2$ und W beispielsweise als Tetrahydropyranyläther geschützt sind, mit p-Toluolsulfonsäurechlorid in das Tosylat der allgemeinen Formel IV überführt.

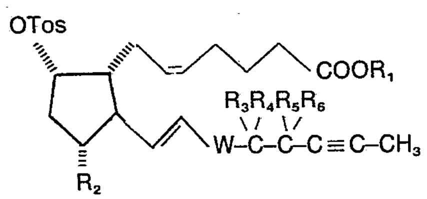
IV

Durch Umsetzung mit Kaliumnitrit in Dimethylsulfoxid erhält man den 9-β-konfigurierten Alkohol V, den man mit p-Toluol-

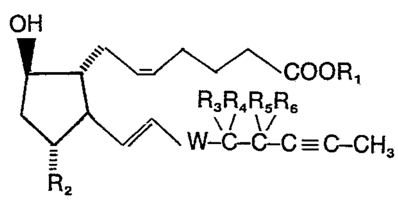
V

sulfonsäurechlorid in Gegenwart von Pyridin zum Tosylat der allgemeinen Formel VI umsetzt.

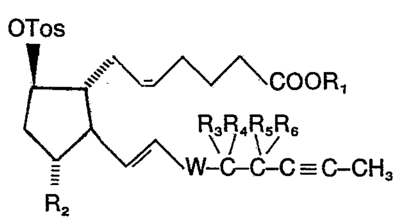
VI

Auf dieser Stufe kann man gegebenenfalls die Tetrahydropyranylätherschutzgruppen abspalten. Das Tosylat wird anschliessend mit Natriumazid in einem polaren, aprotischen Lösungsmittel wie DMF, N-Methylpyrrolidon oder vorzugsweise HMPT(Hexamethylphosphorsäuretriamid) in das Azid der allgemeinen Formel II überführt, welches gegebenenfalls verseift wird ($R_1 = H$).

Die neuen Prostacyclinderivate der allgemeinen Formel I sind wertvolle Pharmaka, da sie bei ähnlichem Wirkungsspektrum gegenüber entsprechenden Prostaglandinen eine bessere Spezifität und vor allem eine wesentlich längere Wirksamkeit besitzen. Im Vergleich zu $PGI_2$ zeichnen sie sich durch grössere Stabilität aus. Die gute Gewebsspezifität der neuen Prostaglandine zeigt sich bei der Untersuchung an glattmuskulären Organen, wie z. B. am Meerschweinchenileum oder an der isolierten Kaninchentrachea, wo eine wesentlich geringere Stimulation zu beobachten ist als bei der Applikation natürlicher Prostaglandine vom E-, A- oder F-Typ. Die neuen Prostaglandin-Analoga besitzen die für Prostacycline typischen Eigenschaften, wie z. B. Senkung des peripheren arteriellen und koronaren vaskulären Widerstandes und damit Senkung des systemischen Blutdrucks, ohne zugleich Schlagvolumen und koronare Durchblutung zu senken. Inhibierung der Thrombozytenaggregation und Inhibierung der Bronchokonstriktion, Hemmung der Magensäuresekretion, antiallergische Eigenschaften, Senkung des pulmonaren vaskulären Widerstandes und des pulmonaren Blutdruckes, Forderung der Nierendurchblutung, Erhöhung der zerebralen Durchblutung. Ausserdem besitzen die neuen Prostaglandinanaloga antiproliferative Eigenschaften.

Bei intravenöser Injektion an wachen, hypertonen Ratten in Dosen von 5, 20 und 100 µg/kg Körpergewicht zeigen die erfindungsgemässen Verbindungen eine stärkere blutdrucksenkende und länger anhaltende Wirkung als $PGE_2$ und $PGA_2$, ohne wie $PGE_2$ Durchfälle oder $PGA_2$ kardiale Arrhythmien auszulösen.

Bei intravenöser Injektion an narkotisierten Kaninchen zeigen die erfindungsgemässen Verbindungen im Vergleich zu $PGE_2$ und $PGA_2$ eine stärkere und erheblich länger anhaltende Blutdrucksenkung, ohne dass andere glattmuskuläre Organe oder Organfunktionen beeinflusst werden.

Für die parenterale Verabreichung werden sterile, injizierbare, wässrige oder ölige Lösungen benutzt. Für die orale Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemässen Wirkstoffe sollen in Verbindung mit den in der Galenik bekannten und üblichen Hilfsstoffen z. B. zur Herstellung von Blutdrucksenkern dienen.

Beispiel 1

(13E)-(11R,15S)-11,15-Dihydroxy-9α,6-nitrilo-13-prosten-18-in-säure

Eine Lösung von 365 mg (5Z,13E)-(9S,11R,15S)-9-Azido-11,15-dihydroxy-5,13-prostadien-18-in-säure in 30 ml Essigsäureäthylester rührt man 27 Stunden bei 70–80 °C unter Argon. Anschliessend dampft man im Vakuum ein und chromatographiert den Rückstand an Kieselgel. Mit Methylenchlorid/Isopropanol (1 + 1) erhält man 230 mg der Titelverbindung als viskoses Öl.

IR ($CHCl_3$): 3610, 3400 (breit), 2940, 2862, 1720, 1640, 1023, 1078, 975/cm.

Das Ausgangsmaterial für die obige Titelverbindung wird wie folgt hergestellt:

1a) (5Z,13E)-(9S,11R,15S)-11,15-Bis(tetrahydropyran-2-yloxy)-9-(p-toluolsulfonyloxy)-5,13-prostadien-18-in-säuremethylester

Zu einer Lösung von 2 g (5Z,13E)-(9S,11R,15S)-11,15-Bis-(tetrahydropyran-2-yloxy)-9-hydroxy-5,13-prostadien-18-in-säuremethylester (hergestellt aus der entsprechenden Säure mit ätherischer Diazomethanlösung) in 4 ml Pyridin fügt man bei 0 °C 1,38 g p-Toluolsulfonylchlorid, rührt 6 Stunden bei Raumtemperatur und lässt anschliessend 60 Stunden bei 5 °C stehen. Man verdünnt dann mit Äther, schüttelt nacheinander einmal mit Wasser, einmal mit eiskalter 3%iger Schwefelsäure, einmal mit Wasser, einmal mit 5%iger Natriumbicarbonatlösung, zweimal mit Wasser, trocknet über Magnesiumsulfat und dampft im Vakuum ein. Dabei erhält man 2,45 g der Titelsubstanz als farbloses Öl.

IR: 2960, 2880, 1732, 1605, 1493, 1370, 1178, 977/cm.

1b) (5Z,13E)-(9R,11R,15S)-11,15-Bis-(tetrahydropyran-2-yloxy)-9-hydroxy-5,13-prostadien-18-in-säuremethylester

Zu einer Lösung von 2,4 g des nach Beispiel 1a hergestellten Tosylats in 50 ml Dimethylsulfoxid fügt man 5,1 g Kaliumnitrit und rührt 4 Stunden bei 65 °C. Anschliessend giesst man auf eine 20%ige Natriumchloridlösung, extrahiert 5× mit je 50 ml einer Mischung aus Pentan/Äther (1 + 1), wäscht die organische Phase dreimal mit je 50 ml Wasser, trocknet über Magnesiumsulfat und dampft im Vakuum ein. Nach Chromatographie des Rückstandes an Kieselgel erhält man mit Äther/Pentan (8 + 2), 1,25 g des invertierten Alkohols als farbloses Öl.

IR: 3400, 2950, 1730, 1440, 975/cm.

1c) (5Z,13E)-(9R,11R,15S)-11,15-Bis-(tetrahydropyran-2-yloxy)-9-(p-toluolsulfonyloxy)-5,13-prostadien-18-in-säuremethylester

Zu einer Lösung von 0,9 g des nach Beispiel 1b hergestellten 9-β-Alkohols in 5 ml Pyridin fügt man bei 0 °C 622 mg p-Toluolsulfonylchlorid und rührt 21 Stunden bei Raumtemperatur unter Argon. Anschliessend verdünnt man mit Äther und schüttelt nacheinander mit Wasser, eiskalter 3%iger Schwefelsäure, Wasser, 5%iger Natriumbicarbonatlösung, 3× mit Wasser. Man trocknet über Magnesiumsulfat und dampft im Vakuum ein. Dabei erhält man 1,09 g des Tosylats als farbloses Öl.

IR: 2962, 1732, 1605, 1493, 1370, 975/cm.

1d) (5Z,13E)-(9R,11R,15S)-11,15-Dihydroxy-9-(p-toluol-sulfonyloxy)-5,13-prostadien-18-in-säuremethylester

1,06 g des nach Beispiel 1c hergestellten Tosylats rührt man 20 Stunden mit 30 ml einer Mischung aus Essigsäure/Wasser/Tetrahydrofuran. (65 + 35 + 10) unter Argon. Man dampft im Vakuum und reinigt den Rückstand durch präparative Schichtchromatographie. Mit Äther als Laufmittel erhält man 485 mg der Titelverbindung als farbloses Öl.

IR: 3610, 3430, 2963, 2940, 1730, 1603, 1363, 1178, 975/cm.

1e) (5Z,13E)-(9S,11R,15S)-9-Azido-11,15-dihydroxy-5,13-prostadien-18-in-säuremethylester

Eine Lösung aus 80 mg des nach Beispiel 1d hergestellten Diols in 1,6 ml Hexamethylphosphorsäuretriamid versetzt man mit 10,8 mg Natriumazid und rührt 6 Stunden bei 40 °C. Man versetzt mit 6 ml einer 20%igen Kochsalzlösung, extrahiert 5× mit einer Mischung aus Äther/Pentan (3 + 1), schüttelt die organische Phase 2× mit je 3 ml Wasser, trocknet mit Magnesiumsulfat und dampft im Vakuum ein. Dabei erhält man die Titelverbindung als dünnschichtchromatographisch einheitliches Öl.

IR: 3400, 2960, 2110, 1730, 975/cm.

1f) (5Z,13E)-(9S,11R,15S)-9-Azido-11,15-dihydroxy-5,13-prostadien-18-in-säure

70 mg des nach Beispiel 1e hergestellten Azids versetzt man mit 2 ml einer Lösung aus 50 mg Kaliumhydroxid in 1,65 ml Methanol und 0,35 ml Wasser und rührt 4 Stunden bei Raumtemperatur unter Argon. Anschliessend kühlt man auf 5 °C, säuert mit 10%iger Zitronensäurelösung auf pH 6 an, extrahiert dreimal mit Methylenchlorid, wäscht den organischen Extrakt zweimal mit je 5 ml Sole, trocknet über Magnesiumsulfat und dampft im Vakuum ein. Nach Chromatographie des Rückstandes an Kieselgel erhält man mit Essigester 39 mg der Titelverbindung als farbloses Öl.

IR: 3600, 3400 (breit), 2110, 1715, 1605, 976/cm.

Beispiel 2

(13E)-(11R,15S,16RS)-11,15-Dihydroxy-16-methyl-9α,6-nitrilo-13-prosten-18-in-säure

Eine Lösung von 520 mg (5Z,13E)-(9S,11R,15S,-16RS)-9-Azido-11,15-dihydroxy-16-methyl-5,13-prostadien-18-in-säure in 50 ml Essigsäureäthylester rührt man 26 Stunden bei 70–80 °C unter Argon. Anschliessend dampft man im Vakuum ein und chromatographiert den Rückstand an Kieselgel. Mit Methylenchlorid/Isopropanol (1 + 1) erhält man 380 mg der Titelverbindung als farbloses Öl.

IR: 3600, 3400 (breit), 2942, 2860, 1720, 1640, 1025, 1080, 976/cm.

Das Ausgangsmaterial für die obige Titelverbindung wird wie folgt hergestellt:

2a) (5Z,13E)-(9S,11R,15S,16RS)-11,15-Bis-(tetrahydropyran-2-yloxy)-16-methyl-9-(p-toluolsulfonyloxy)-5,13-prostadien-18-in-säure-methylester

Zu einer Lösung von 3,05 g (5Z,13E)-(9S,11R,-15S,16RS)-11,15-Bis-(tetrahydropyran-2-yloxy)-9-hydroxy-16-methyl-5,13-prostadien-18-insäure-methylester (hergestellt aus der entsprechenden Säure mit ätherischer Diazomethanlösung) in 6 ml Pyridin fügt man bei 0 °C 2,15 g p-Toluolsulfonylchlorid, rührt 6 Stunden bei Raumtemperatur und

lässt anschliessend 60 Stunden bei 5 °C stehen. Man verdünnt mit Äther, schüttelt nacheinander 1 × mit Wasser, 1 × mit eiskalter 3%iger Schwefelsäure, 1 × mit Wasser, 1 × mit 5%iger Natriumbicarbonatlösung, 2 × mit Wasser, trocknet über Magnesiumsulfat und dampft im Vakuum ein. Dabei erhält man 3,7 g des Tosylats als farbloses Öl.

IR: 2960, 2878, 1733, 1605, 1492, 1370, 1178, 975/cm.

2b) (5Z,13E)-(9R,11R,15S,16RS)-11,15-Bis-(tetrahydropyran-2-yloxy)-9-hydroxy-16-methyl-5,13-prostadien-18-in-säuremethylester

Zu einer Lösung von 3,6 g des nach Beispiel 2a hergestellten Tosylats in 70 ml Dimethylsulfoxid fügt man 6,8 g Kaliumnitrit und rührt 4 Stunden bei 65 °C unter Argon. Anschliessend giesst man auf eine 20%ige Natriumchloridlösung, extrahiert 5 × mit je 70 ml einer Mischung aus Pentan/Äther (1 + 1), wäscht die organische Phase dreimal mit je 60 ml Wasser, trocknet über Magnesiumsulfat und dampft im Vakuum ein. Nach Chromatographie des Rückstandes an Kieselgel erhält man mit Äther/Pentan (8 + 2) 2,5 g des invertierten Alkohols als farbloses Öl.

IR: 3410, 2950, 1732, 1440, 976/cm.

2c) (5Z,13E)-(9R,11R,15S,16RS)-11,15-Bis-(tetrahydropyran-2-yloxy)-16-methyl-9-(p-toluolsulfonyloxy)-5,13-prostadien-18-in-säuremethylester

Eine Lösung von 2,5 g des nach Beispiel 2b hergestellten 9β-Alkohols in 15 ml Pyridin versetzt man bei 0 °C mit 1,7 g p-Toluolsulfonylchlorid und lässt 24 Stunden bei Raumtemperatur stehen. Man versetzt mit 0,5 ml Wasser und lässt eine weitere Stunde bei Raumtemperatur stehen. Anschliessend verdünnt man mit Äther, schüttelt nacheinander mit eiskalter 3%iger Schwefelsäure, Natriumbicarbonatlösung und Wasser, trocknet über Magnesiumsulfat und dampft im Vakuum ein. Man erhält 3 g des 9β-Tosylats als Öl.

IR: 2955, 1735, 1605, 1492, 1370, 975/cm.

2d) (5Z,13E)-(9R,11R,15S,16RS)-11,15-Dihydroxy-16-methyl-9-(p-toluolsulfonyloxy)-5,13-prostadien-18-in-säuremethylester

2,90 g des nach Beispiel 2c hergestellten 9β-Tosylats rührt man 24 Stunden mit 80 ml einer Mischung aus Essigsäure/Wasser/Tetrahydrofuran (65/35/10), dampft im Vakuum ein und reinigt den Rückstand durch Chromatographie über Kieselgel mit Hexan/Essigester-Gemischen. Dabei erhält man 1,40 g des 11,15-Diols als farbloses Öl.

IR: 3600, 3400, 2958, 1735, 1605, 1368, 978/cm.

2e) (5Z,13E)-(9S,11R,15S,16RS)-9-Azido-11,15-dihydroxy-16-methyl-5,13-prostadien-18-in-säuremethylester

1 g des nach Beispiel 2d hergestellten Diols in 20 ml Hexamethylphosphorsäuretriamid und 130 mg Natriumazid werden 6 Stunden bei 40 °C gerührt. Man kühlt ab, versetzt mit 100 ml Kochsalzlösung und extrahiert mehrmals mit Äther/Pentan (3 + 1), wäscht die organische Phase mit Wasser, trocknet über Magnesiumsulfat und dampft im Vakuum ein. Man erhält die 9-Azidoverbindung als viskoses Öl.

IR: 3600, 3420, 2958, 2110, 1735, 978/cm.

2f) (5Z,13E)-(9S,11R,15S,16RS)-9-Azido-11,15-dihydroxy-16-methyl-5,13-prostadien-18-in-säure

200 mg des nach Beispiel 2e hergestellten Azids werden mit methanolischer Kalilauge wie in Beispiel 1f behandelt. Das Rohprodukt wird über Kieselgel mit Essigester chromatographiert. Man erhält 150 mg der Säure als farbloses Öl.

IR: 3600, 3410, 2952, 2110, 1710, 978/cm.

Beispiel 3

(13E)-(11R,15R)-11,15-Dihydroxy-16,16-dimethyl-9α,6-nitrilo-13-prosten-18-in-säure

Eine Lösung von 300 mg (5Z,13E)-(9S,11R,15R)-9-Azido-11,15-dihydroxy-16,16-dimethyl-5,13-prostadien-18-in-säure in 25 ml Essigsäureäthylester erhitzt man 24 Stunden auf 70–75 °C unter Argon. Nach Verdampfen des Lösungsmittels im Vakuum chromatographiert man den Rückstand an Kieselgel. Mit Methylenchlorid/10–30% Isopropanol erhielt man 200 mg der Titelverbindung als Öl.

IR: 3600, 3400 (breit), 2950, 2860, 1715, 1640, 1020, 978/cm.

Das Ausgangsmaterial für die obige Titelverbindung wird wie folgt hergestellt:

3a) (5Z,13E)-(9S,11R,15R)-11,15-Bis-(tetrahydropyran-2-yloxy)-16,16-dimethyl-9-(p-toluolsulfonyloxy)-5,13-prostadien-18-in-säuremethylester

3 g (5Z,13E)-(9S,11R,15R)-11,15-Bis-(tetrahydropyran-2-yloxy)-16,16-dimethyl-9-hydroxy-5,13-prostadien-18-in-säuremethylester (hergestellt aus der Carbonsäure mit ätherischer Diazomethanlösung), gelöst in 10 ml Pyridin, versetzt man bei 0 °C mit 2,09 g p-Toluolsulfonylchlorid und rührt anschliessend 48 Stunden bei +5 °C. Man versetzt mit 0,5 ml Wasser, rührt eine weitere Stunde, verdünnt mit 200 ml Äther und wäscht nacheinander mit eiskalter 10%iger Schwefelsäure, Natriumbicarbonatlösung und Wasser, trocknet über Magnesiumsulfat und dampft im Vakuum ein. Man erhält 4 g des 9α-Tosylats, das ohne weitere Reinigung weiter verwendet wird.

IR: 2960, 2868, 1735, 1602, 1360, 1175, 975/cm.

3b) (5Z,13E)-(9R,11R,15R)-11,15-Bis-(tetrahydropyran-2-yloxy)-16,16-dimethyl-9-hydroxy-5,13-prostadien-18-in-säuremethylester

3,8 g des nach Beispiel 3a erhaltenen Tosylats und 7,6 g Kaliumnitrit werden mit 80 ml Dimethylsulfoxid 4 Stunden bei 65 °C gerührt, man verdünnt dann mit Sole, extrahiert mehrmals mit Äther/Pentan-Mischung (1 + 1), wäscht die organische Phase mit Sole, trocknet über Magnesiumsulfat und dampft im Vakuum ein. Nach Chromatographie über Kieselgel mit Hexan/Essigester-Gradienten erhält man 1,65 g des 9β-Alkohols als farbloses Öl.

IR: 3540, 2950, 2860, 1735, 980/cm.

3c) (5Z,13E)-(9R,11R,15R)-11,15-Bis-(tetrahydropyran-2-yloxy)-16,16-dimethyl-9-(p-toluolsulfonyloxy)-5,13-pro-stadien-18-in-säuremethylester

Eine Lösung von 1,50 g des nach Beispiel 3b erhaltenen 9β-Alkohols in 10 ml Pyridin wird bei 0 °C mit 1,045 g p-Toluolsulfonylchlorid versetzt, rührt 20 Stunden bei Raumtemperatur, versetzt mit 0,2 ml Wasser und arbeitet wie in Beispiel 3a beschrieben auf. Man erhält 2,1 g des 9β-Tosylats als Öl.

IR: 2960, 2860, 1735, 1601, 1365, 1175, 980/cm.

3d) (5Z,13E)-(9R,11R,15R)-11,15-Dihydroxy-16,16-dimethyl-9-(p-toluol-sulfonyloxy)-5,13-prostadien-18-in-säuremethylester

2 g des nach Beispiel 3c erhaltenen 9β-Tosylats werden zur Abspaltung der Tetrahydropyranylätherschutzgruppen mit Essigsäure analog Beispiel 1d behandelt.

Nach Reinigung über Kieselgel mit Hexan/Essigester-Gradienten erhält man 1,1 g des 11,15-Diols als Öl.

IR: 3600, 3420, 2955, 1735, 1601, 1360, 1175, 978/cm.

3e) (5Z,13E)-(9S,11R,15R)-9-Azido-11,15-dihydroxy-16,16-dimethyl-5,13-prostadien-18-in-säuremethylester

Eine Lösung von 1,1 g der nach Beispiel 3d hergestellten Verbindung in 20 ml Hexamethylphosphorsäuretriamid wird mit 150 mg Natriumazid 6 Stunden auf 40 °C erhitzt. Man verdünnt mit 150 ml Sole, schüttelt mehrmals mit Äther/Pentan-Mischung (3:1) aus, wäscht die organische Phase mit Wasser, trocknet über Magnesiumsulfat und dampft im Vakuum ein. Das Rohprodukt reinigt man über Kieselgel mit Hexan/Essigester-Gemischen und erhält 700 mg des 9α-Azids als Öl.

IR: 3520, 2960, 2110, 1735, 978/cm.

3f) (5Z,13E)-(9S,11R,15R)-9-Azido-11,15-dihydroxy-16,16-dimethyl-5,13-prostadien-18-in-säure

500 mg der nach Beispiel 3e erhaltenen Verbindung werden analog Beispiel 1f verseift. Man erhält 400 mg der oben angegebenen Carbonsäure als farbloses Öl.

IR: 3600, 3400 (breit), 2955, 2110, 1712, 978/cm.

Beispiel 4

(13E)-(11R,15RS)-11,15-Dihydroxy-15-methyl-9α,6-nitrilo-13-prosten-18-in-säure

200 mg (5S,13E)-(9S,11R,15RS)-9-Azido-11,15-dihydroxy-15-methyl-5,13-prostadien-18-in-säure werden in 20 ml Essigsäureäthylester 24 Stunden auf 70 °C erhitzt. Nach Verdampfen des Lösungsmittels chromatographiert man an Kieselgel mit Methylenchlorid/10–30% Isopropanol und erhält 120 mg der Titelverbindung als Öl.

IR: 3600, 3400, 2950, 2845, 1712, 1640, 978/cm.

Das Ausgangsmaterial für die obige Titelverbindung wird wie folgt hergestellt:

4a) (5Z,13E)-(9S,11R,15RS)-11,15-Bis-(tetrahydropyran-2-yloxy)-15-methyl-9-(p-toluolsulfonyloxy)-5,13-prostadien-18-in-säuremethylester

2 g (5Z,13E)-(9S,11R,15RS)-11,15-Bis-(tetrahydropyran-2-yloxy)-9-hydroxy-15-methyl-5,13-prostadien-18-in-säure-methylester (hergestellt aus der Carbonsäure mit Diazomethan) werden in Analogie zu Beispiel 1a in 2,3 g öliges 9α-Tosylat überführt.

IR: 2960, 2865, 1735, 1601, 1365, 1175, 975/cm.

4b) (5Z,13E)-(9R,11R,15RS)-11,15-Bis-(tetrahydropyran-2-yloxy)-9-hydroxy-15-methyl-5,13-prostadien-18-in-säuremethylester

2,2 g des nach Beispiel 4a hergestellten Tosylats werden in Analogie zu Beispiel 1b mit Kaliumnitrit umgesetzt. Man erhält 1,3 g des 9β-Alkohols als farbloses Öl.

IR: 3450, 2955, 1735, 978/cm.

4c) (5Z,13E)-(9R,11R,15RS)-11,15-Bis-(tetrahydropyran-2-yloxy)-15-methyl-9-(p-Toluolsulfonyloxy)-5,13-prostadien-18-in-säuremethylester

In Analogie zu Beispiel 1c erhält man aus 1,3 g des nach Beispiel 4b hergestellten 9β-Alkohols 1,7 g des 9β-Tosylats als Öl.

IR: 2960, 2860, 1735, 1601, 1370, 1175, 978/cm.

4d) (5Z,13E)-(9R,11R,15RS)-11,15-Dihydroxy-15-methyl-9-(p-toluolsulfonyloxy)-5,13-prostadien-18-in-säuremethylester

In Analogie zu Beispiel 1d erhält man aus 1,7 g der nach Beispiel 4c hergestellten Substanz 1 g des 11,15-Diols als farbloses Öl.

IR: 3600, 3450, 2960, 1735, 1601, 1365, 1178, 975/cm.

4e) (5Z,13E)-(9S,11R,15RS)-9-Azido-11,15-dihydroxy-15-methyl-5,13-prostadien-18-in-säuremethylester

1 g der nach Beispiel 4d hergestellten Verbindung wird in Analogie zu Beispiel 1c mit Natriumazid umgesetzt. Man erhält 600 mg der 9-Azidoverbindung als farbloses Öl.

IR: 3600, 2955, 2110, 1735, 975/cm.

4f) (5Z,13E)-(9S,11R,15RS)-9-Azido-11,15-dihydroxy-15-methyl-5,13-prostadien-18-in-säure

600 mg der nach Beispiel 4e hergestellten Verbindung werden in Analogie zu Beispiel 1f verseift, man erhält 500 mg der Carbonsäure als Öl.

IR: 3600, 3420 (breit), 2960, 2110, 1710, 975/cm.

Beispiel 5

(13E)-(11R,15S)-11,15-Dihydroxy-9α,6-nitrilo-13-prosten-18-in-säuremethylester

Eine Lösung von 150 mg (13E)-(11R,15S)-11,15-Dihydroxy-9α-nitrilo-13-prosten-18-in-säure (siehe Beispiel 1) in 10 ml Methylenchlorid versetzt man bei −10 °C tropfenweise mit einer ätherischen Diazomethanlösung bis zur bleibenden Gelbfärbung, dampft im Vakuum ein und reinigt den Rückstand durch präparative Schichtchromatographie an Kieselgelplatten mit Essigester/Methanol (9+1) als Laufmittel. Man erhält 120 mg der Titelverbindung als farbloses Öl.

IR: 3450, 2960, 1735, 1642, 978/cm.

Beispiel 6

(13E)-(11R,15S,16RS)-11,15-Dihydroxy-16-methyl-9α,6-nitrilo-13-prosten-18-in-säure-methylester

In Analogie zu Beispiel 5 erhält man aus (13E)-(11R,15S,16RS)-11,15-Dihydroxy-16-methyl-9α,6-nitrilo-13-prosten-18-in-säure (siehe Beispiel 2) die Titelverbindung als Öl.

IR: 3450, 2955, 1735, 1640, 978/cm.

Beispiel 7

(13E)-(11R,15R)-11,15-Dihydroxy-16,16-dimethyl-9α,6-nitrilo-13-prosten-18-in-säure-methylester

In Analogie zu Beispiel 5 erhält man aus (13E)-(11R,15R)-11,15-Dihydroxy-(16,16-dimethyl-9α,6-nitrilo-13-prosten-18-in-säure (siehe Beispiel 3) die Titelverbindung als Öl.

IR: 3420, 2955, 1735, 1640, 975/cm.

Beispiel 8

(13E)-(11R,15RS)-11,15-Dihydroxy-15-methyl-9α,6-nitrilo-13-prosten-18-in-säuremethylester

In Analogie zu Beispiel 5 erhält man aus (13E)-(11R,15RS)-11,15-Dihydroxy-15-methyl-9α,6-nitrilo-13-prosten-18-in-säure (siehe Beispiel 4) die Titelverbindung als Öl.

IR: 3450, 2955, 1735, 1640, 978/cm.

Beispiel 9

Tris-(hydroxymethyl)-aminomethansalz von (13E)-(11R,15S,16RS)-11,15-Dihydroxy-16-methyl-9α,6-nitrilo-13-prosten-18-in-säure

Zu einer Lösung von 181 mg (13E)-(11R,15S,-16RS)-11,15-Dihydroxy-16-methyl-9α,6-nitrilo-13-prosten-18-in-säure (siehe Beispiel 2) in 30 ml Acetonitril gibt man bei 65 °C eine Lösung von 62 mg Tris(hydroxymethyl)-aminomethan in 0,2 ml Wasser. Unter Rühren lässt man abkühlen, dekantiert nach 16 Stunden und trocknet den Rückstand bei 25 °C/0,1 Torr. Dabei erhält man 160 mg der Titelverbindung als wachsartige Masse.

Beispiel 10

(13E)-(11R,15S,16RS)-11,15-Dihydroxy-16-methyl-9α,6-nitrilo-13-prosten-18-in-säurebutylester

In Analogie zu Beispiel 5 erhält man aus der nach Beispiel 2 hergestellten Säure mit Diazobutan die Titelverbindung als Öl.

IR: 3430 (breit), 2960, 1737, 1640, 977/cm.

**Patentansprüche**

1. Prostanderivate der allgemeinen Formel I

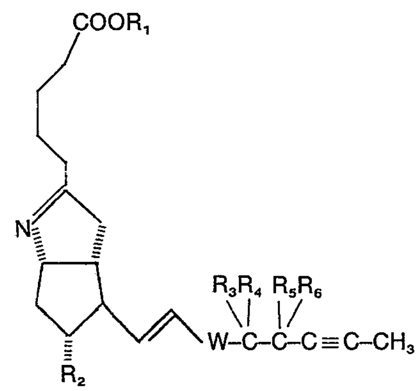

I

worin

$R_1$ Wasserstoff, Alkyl mit 1–4 C-Atomen, Cycloalkyl mit 5 oder 6 C-Atomen oder Phenyl,

W eine freie oder durch Tetrahydropyranyl substituierte Hydroxymethylengruppe oder eine freie oder durch Tetrahydropyranylsubstituierte 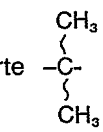 Gruppe, wobei die OH-Gruppe α-ständig ist und $R_3$, $R_4$, $R_5$, $R_6$ ein Wasserstoffatom oder eine Alkylgruppe mit 1–5 C-Atomen,

$R_2$ eine freie oder durch Tetrahydropyranyl substituierte Hydroxygruppe und falls $R_1$ die Bedeutung eines Wasserstoffatoms hat, deren Salze mit physiologisch verträglichen Basen bedeuten.

2. (13E)-(11R,15S,16RS)-11,15-Dihydroxy-16-methyl-9α,6-nitrilo-13-prosten-18-in-säure.

3. Tris-(hydroxymethyl)-aminomethansalz von (13E)-(11R,15S,16RS)-11,15-Dihydroxy-16-methyl-9α,6-nitrilo-13-prosten-18-in-säure.

4. Verfahren zur Herstellung von Prostanderivaten der allgemeinen Formel I, dadurch gekennzeichnet, dass man in an sich bekannter Weise eine Verbindung der allgemeinen Formel II,

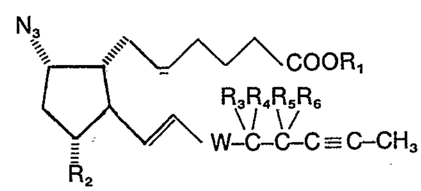

II

worin $R_1$, $R_2$, $R_3$, $R_4$, $R_5$, $R_6$ und W die obengenannten Bedeutungen haben, in einem inerten Lösungsmittel einer thermischen Behandlung unterwirft und gegebenenfalls anschliessend in beliebiger Reihenfolge geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe mit einer physiologisch verträglichen Base in ein Salz überführt.

5. Arzneimittel, bestehend aus einer oder mehreren Verbindungen gemäss Anspruch 1 und üblichen Hilfs- und Trägerstoffen.

**Claims**

1. Prostane derivatives of the general formula I

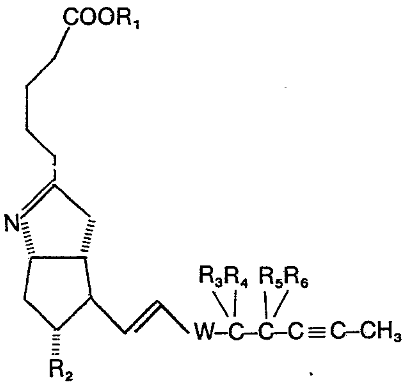

I

in which

$R_1$ represents hydrogen, alkyl having from 1 to 4 carbon atoms, cycloalkyl having 5 or 6 carbon atoms, or phenyl,

W represents a free hydroxymethylene group or a hydroxymethylene group substituted by tetrahydropyranyl or represents a free 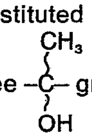 group or a 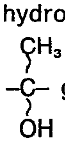 group substituted by tetrahydropyranyl, the OH group being in the α-configuration, and

$R_3$, $R_4$, $R_5$ and $R_6$ represent a hydrogen atom or an alkyl group having from 1 to 5 carbon atoms, and

$R_2$ represents a free hydroxy group or a hydroxy group substituted by tetrahydropyranyl, and, if $R_1$ represents a hydrogen atom, salts thereof with physiologically acceptable bases.

2. (13E)-(11R,15S,16RS)-11,15-dihydroxy-16-methyl-9α,6-nitrilo-13-prosten-18-ynoic acid.

3. Tris-(hydroxymethyl)-aminomethane salt of (13E)-(11R,15S,16RS)-11,15-dihydroxy-16-methyl-9α,6-nitrilo-13-prosten-18-ynoic acid.

4. Process for the manufacture of prostane derivatives of the general formula I, characterised in that in a manner known per se a compound of the general formula II,

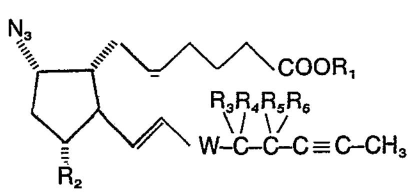

II

in which $R_1$, $R_2$, $R_3$, $R_4$, $R_5$, $R_6$ and W have the abovementioned meanings, is subjected to thermal treatment in an inert solvent and then optionally and in any order protected hydroxy groups are freed and/or free hydroxy groups are esterified or etherified and/or a free carboxyl group is esterified and/or an esterified carboxyl group is saponified or a carboxyl group is converted into a salt with a physiologically acceptable base.

5. Medicaments comprising one or more compounds according to claim 1 and customary auxiliaries and carriers.

**Revendications**

1. Dérivés du prostane répondant à la formule générale I

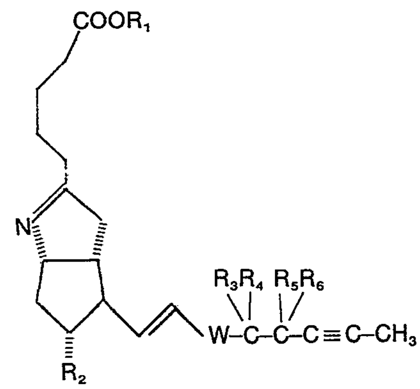

I

dans laquelle

$R_1$ représente l'hydrogène, un alkyle en $C_1$–$C_4$, un cycloalkyle en $C_5$ ou $C_6$ ou un phényle,

W représente un radical hydroxy-méthylène dont le groupe hydroxy est libre ou est éthérifié par un radical tétrahydroxy-pyrannyle, ou représente un radical 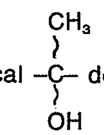 dont le groupe hydroxy est libre ou est éthérifié par un radical tétrahydropyrannyle, le groupe OH ayant la configuration α,

$R_3$, $R_4$, $R_5$ et $R_6$ représentent chacun un atome d'hydrogène ou un radical alkyle en $C_1$–$C_5$ et

$R_2$ représente un groupe hydroxy libre ou sous la forme d'un éther tétrahydropyrannylique, et également, dans le cas où $R_1$ représente un atome d'hydrogène, leurs sels avec des bases acceptables du point de vue physiologique.

2. Acide dihydroxy-11,15 méthyl-16 nitrilo-9α,6 prostène-13 yne-18 oïque-(13E)-(11R,15S,16RS).

3. Sel formé par le tris-(hydroxyméthyl)-aminométhane et l'acide dihydroxy-11,15 méthyl-16 nitrilo-9α,6 prostène-13 yne-18 oïque-(13E)-(11R,15S,16RS).

4. Procédé de préparation de dérivés du prostane de formule générale I, procédé caractérisé en ce qu'on soumet un composé répondant à la formule générale II

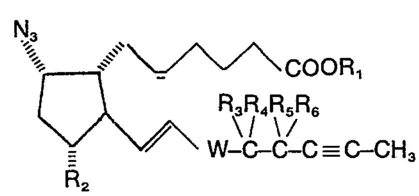

II

dans laquelle $R_1$, $R_2$, $R_3$, $R_4$, $R_5$, $R_6$ et W ont les significations données à la revendication 1, dans un solvant inerte, à un traitement par la chaleur, de manière connue, et ensuite, le cas échéant, dans l'ordre que l'on veut, on libère des groupes hydroxy protégés et/ou on estérifie ou éthérifie des groupes hydroxy libres et/ou on estérifie un groupe carboxy libre et/ou on saponifie un groupe carboxy estérifié ou on transforme un groupe carboxy, avec une base acceptable du point de vue physiologique, en un sel.

5. Médicament constitué d'un ou plusieurs composés selon la revendication 1 ainsi que d'adjuvants et d'excipients usuels.